# FASCICULE DE BREVET EUROPEEN

(11) **EP 0 655 236 B1**
(45) Date de publication et mention de la délivrance du brevet: **07.10.1998**
(21) Numéro de dépôt: 94402651.7
(22) Date de dépôt: 21.11.1994
(51) Int. Cl.: A61K 7/50, A61K 7/06, A61K 7/48

(54) **Composition fondante contenant des sels d'ammonium quaternaire à fonction ester, son utilisation comme produit capillaire**
Quaternäre Ammoniumsalze mit Esterfunktion enthaltende schmelzbare Zusammensetzung und Verwendung in Haarpflegemitteln
Meltable composition containing quaternary ammonium salts with ester function, use in hair care products

(30) Priorité: 26.11.1993 FR 9314155
(43) Date de publication de la demande: 31.05.1995
(73) Titulaire: L'OREAL, 75008 Paris (FR)
(72) Inventeur: Cervantes, Frédéric, F-75017 Paris (FR); Sebag, Henri, F-75016 Paris (FR)
(74) Mandataire: Andral, Christophe André Louis

(56) Documents cités:
- EP-A- 0 181 547
- EP-A- 0 252 441
- EP-A- 0 282 864
- EP-A- 0 309 052
- EP-A- 0 384 415
- FR-A- 2 356 627
- US-A- 4 269 824

## Description

La présente invention se rapporte à une composition aqueuse contenant des sels d'ammonium quaternaire à fonction ester utilisables en particulier dans le domaine cosmétique. Cette composition est plus spécialement destinée au domaine capillaire comme produit de soin et/ou de traitement éventuellement suivi d'un rinçage à l'eau. L'invention se rapporte aussi à l'utilisation de cette composition et à un procédé pour le traitement et/ou le soin des cheveux

Il est bien connu que les cheveux sont généralement sensibilisés à des degrés divers par l'action des agents atmosphériques ainsi que par l'action des traitements mécaniques ou chimiques tels que les colorations, les décolorations et/ou les permanentes, de sorte que les cheveux sont souvent difficiles à démêler et à coiffer.

L'un des moyens couramment utilisés pour améliorer le démêlage et la douceur de ces cheveux consiste à utiliser des compositions de soin, puis à rincer les cheveux à l'eau. En général, ces compositions sont employées après un shampooing éventuellement précédé par l'un des traitements ci-dessus.

Les produits de soin capillaire, recherchés par les utilisateurs sont ceux que l'on peut doser et prendre aisément dans la main. Pour ce faire, ces produits doivent présenter une certaine consistance. En effet, un produit liquide est beaucoup plus difficile à doser et s'écoule facilement entre les doigts. En revanche, il s'applique mieux sur les cheveux et se répartit de façon homogène. Malheureusement, les produits épais sont généralement difficiles à étaler sur les cheveux et confèrent un soin non homogène de la chevelure.

Certains produits de soin capillaire ont la particularité d'être "fondant". Autrement dit, ces produits sont, au départ, relativement épais et facilement préhensibles, et au fur et à mesure de leur manipulation avec les mains et de leur application sur les cheveux, ils se fluidifient de façon irréversible jusqu'à présenter une viscosité proche de celle de l'eau. Cette fluidification assure une très bonne répartition du produit sur l'ensemble de la chevelure. La simple agitation ou élévation de température ne permet pas la fluidification du produit.

Une des compositions présentant ce caractère "fondant" particulièrement original, est une dispersion aqueuse de 10 à 15% en poids, par rapport au poids total de la composition, de chlorure de diméthyl distéaryl ammonium qui est un tensioactif cationique couramment utilisé. Cette composition présente, à température ambiante et avant manipulation, une viscosité de 4 Pa.s pour une concentration en sel d'ammonium de 10% et une viscosité de 7,6 Pa.s pour 15% de ce même sel.

Malheureusement, ce tensioactif cationique présente l'inconvénient d'être insuffisamment biodégradable. Or, vu les préoccupations actuelles en faveur de l'environnement, on recherche de plus en plus des produits biodégradables.

Il existe donc le besoin d'une composition présentant une meilleure biodégradabilité et ayant le caractère "fondant" décrit plus haut.

On sait que les esters et en particulier les sels d'ammonium quaternaire à fonction ester ont l'avantage d'être biodégradables ( Cf, A new generation of softeners : Tenside Surf. Det. 30 (1993) 3 p.186-191 ). Malheureusement, la dispersion de ces sels d'ammonium à fonction ester est liquide à température ambiante. Aussi, il est nécessaire d'épaissir cette dispersion pour obtenir une composition ayant une certaine consistance.

Dans le domaine cosmétique, il est classique d'utiliser comme épaississant d'une composition aqueuse des produits solides et en particulier des alcools gras solides tels que les alcools cétylique, stéarylique ou leurs mélanges qui ont l'avantage d'être biodégradables. Ainsi, la demanderesse a réalisé des compositions aqueuses épaisses en associant ces alcools gras solides à des sels d'ammonium à fonction ester. Malheureusement, ces compositions ne présentaient pas le caractère fondant recherché.

Aussi, la demanderesse a cherché d'autres produits susceptibles d'épaissir les sels d'ammonium quaternaire à fonction ester et en particulier des produits biodégradables.

De façon surprenante, la demanderesse a trouvé que l'association d'un sel d'ammonium quaternaire à fonction ester biodégradable à certains corps gras liquides permettait l'obtention d'une composition à caractère fondant présentant une viscosité suffisante avant toute manipulation.

De façon plus précise, l'invention a pour objet une composition caractérisée en ce qu'elle comprend dans un milieu aqueux, au moins un sel d'ammonium quaternaire comportant au moins une fonction ester, au moins un corps gras liquide à une température au plus égale à 40°C, le corps gras et les concentrations en sel d'ammonium et en corps gras étant choisis de façon que la composition ait une viscosité au moins égale à 0,6 Pa.s, au repos et avant utilisation et en ce qu'elle présente un caractère fondant.

Dans la suite du texte, on notera les corps gras liquides à au plus 40°C par corps gras "liquides".

Il est décrit dans les documents EP-A-309052, EP-A-415698 et EP-A-525271 l'association entre autres d'un sel d'ammonium quaternaire à fonction ester et d'un alcool gras oxyéthyléné dans des compositions adoucissantes liquides pour textiles. Mais ces compositions ont une viscosité insuffisante et/ou ne présentent pas le caractère fondant.

Dans la composition selon l'invention, on peut utiliser un ou plusieurs sels d'ammonium à fonction ester comportant une, deux ou trois fonctions ester et un ou plusieurs corps gras "liquides".

La viscosité de la composition de l'invention est choisie inférieure à 12 Pa.s et de préférence allant de 1 Pa.s à 6 Pa.s.

Le sel d'ammonium quaternaire contenant au moins une fonction ester utilisable dans l'invention peut présenter la formule (I) suivante : 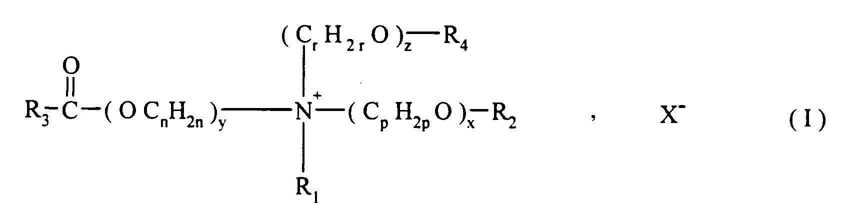 dans laquelle :
R₁ est choisi parmi les radicaux alkyles en C₁-C₆ et les radicaux hydroxyalkyles ou dihydroxyalkyles en C₁-C₆ ;
R₂ est choisi parmi :
   - le radical 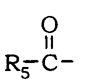
   - les radicaux R₆ hydrocarbonés en C₁-C₂₂ linéaires ou ramifiés, saturés ou insaturés,
   - l'atome d'hydrogène,
R₄ est choisi parmi :
   - le radical 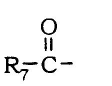
   - les radicaux R₈ hydrocarbonés en C₁-C₆ linéaires ou ramifiés, saturés ou insaturés,
   - l'atome d'hydrogène,
R₃, R₅ et R₇, identiques ou différents, sont choisis parmi les radicaux hydrocarbonés en C₇-C₂₁, linéaires ou ramifiés, saturés ou insaturés ;
n, p et r, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ;
à condition que la somme x + y + z vaille de 1 à 15 , que lorsque x vaut 0 alors R₂ désigne R₆ et que lorsque z vaut 0 alors R₄ désigne R₈.

Les radicaux alkyles R₁ peuvent être linéaires ou ramifiés et plus particulièrement linéaires.

De préférence R₁ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₂ est un radical R₆ hydrocarboné, il peut être long et avoir de 12 à 22 atomes de carbone ou court et avoir de 1 à 3 atomes de carbone.

Lorsque R₄ est un radical R₈ hydrocarboné, il a de préférence 1 à 3 atomes de carbone.

Avantageusement, R₃, R₅ et R₇, identiques ou différents, sont choisis parmi les radicaux hydrocarbonés en C₁₁-C₂₁, linéaires ou ramifiés, saturés ou insaturés.

De préférence, x et z, identiques ou différents, valent 0 ou 1.

Avantageusement, y est égal à 1.

De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement les sels d'ammonium de formule ( I ) dans laquelle :
R₁ désigne un radical méthyle ou éthyle,
x et y sont égaux à 1 ;
z est égal à 0 ou 1 ;
n, p et r sont égaux à 2 ;
R₂ est choisi parmi :
   - le radical 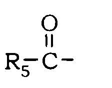
   - les radicaux méthyle, éthyle ou hydrocarbonés en C₁₄-C₂₂
   - l'atome d'hydrogène ;
R₄ est choisi parmi :
   - le radical 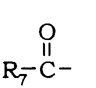
   - l'atome d'hydrogène ;
R₃, R₅ et R₇, identiques ou différents, sont choisis parmi les radicaux hydrocarbonés en C₁₃-C₁₇, linéaires ou ramifiés, saturés ou insaturés.

Avantageusement, les radicaux hydrocarbonés sont linéaires.

On peut citer par exemple les composés de formule ( I ) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées et d'acides gras ou de mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple vendus sous les dénominations DEHYQUART par la société HENKEL, STEPANQUAT par la société STEPAN, NOXAMIUM par la société CECA, REWOQUAT WE 18 par la société REWO-WITCO.

La composition selon l'invention contient de préférence un mélange de sels de mono, di et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl dihydroxyéthyl méthyl ammonium, 45 à 60% de méthylsulfate de diacyloxyéthyl hydroxyéthyl méthyl ammonium et 15 à 30% de méthylsulfate de triacyloxyéthyl méthyl ammonium, les radicaux acyles ayant de 14 à 18 atomes de carbone et provenant d'huile de palme éventuellement partiellement hydrogénée.

On peut aussi utiliser les sels d'ammonium décrits dans le brevet US-A-4874554.

On peut aussi utiliser des sels d'ammonium ne répondant pas à la formule ( I ) et par exemple ceux décrits dans le document US-A-4137180.

Le sel d'ammonium quaternaire à fonction ester est présent en pratique dans des quantités allant de 5% à 40% en poids par rapport au poids total de la composition et de préférence de 8% à 20% en poids.

Selon l'invention, le corps gras liquide à une température au plus égale à 40°C est un composé qui peut être choisi parmi les alcools gras éventuellement oxyalkylénés ou polyglycérolés et les poly a-oléfines éventuellement hydrogénées et leurs mélanges. En particulier, ce corps gras peut être solide à température ambiante (20°C) et être liquide avant 40°C. La température de 40°C correspond en fait à la température maximale de l'eau généralement utilisée lors de l'application de produits capillaires.

Les alcools gras "liquides" sont choisis parmi les alcools gras en C₈-C₂₂, linéaires ou ramifiés ; ils sont éventuellement oxyalkylénés avec 1 à 15 moles d'oxyde d'alkylène ou polyglycérolés avec 1 à 6 moles de glycérol. L'oxyde d'alkylène est de préférence l'oxyde d'éthylène et/ou de propylène.

Plus particulièrement, les alcools gras "liquides" sont choisis parmi les alcools laurique, myristique, isostéarylique, isocétylique et oléique, les alcools lauriques oxyéthylénés de 2 à 8 moles d'oxyde d'éthylène et leurs mélanges.

Les poly α-oléfines sont choisies notamment parmi les polybutènes et les polydécènes éventuellement hydrogénés et leurs mélanges.

Un polybutène particulièrement utilisable dans l'invention est l'heptaméthylnonane vendu sous la dénomination ARLAMOL HD par la société ICI. On peut également utiliser les polybutènes avec un nombre de motifs au plus égal à 8 vendus sous les dénominations PERMETHYL par la société PRESPERSE.

En pratique, le corps gras "liquide" est présent dans des quantités allant de 1% à 15% en poids par rapport au poids total de la composition et de préférence de 2 à 12% en poids.

La composition de l'invention peut également contenir au moins un additif choisi parmi les alcools gras solides à plus de 40°C, d'autres épaississants, les esters d'acides gras, les esters d'acides gras et de glycérol, les silicones (volatiles ou non, fonctionnalisés ou non), les tensioactifs cationiques autres que les sels d'ammonium à fonction ester, les parfums, les conservateurs, les filtres solaires, les protéines, les vitamines, les polymères, les huiles végétales, animales minérales ou synthétiques et tout autre additif classiquement utilisé dans le domaine cosmétique.

Ces additifs sont présents dans la composition selon l'invention dans des proportions allant de 0 à 20% en poids par rapport au poids total de la composition. La quantité précise de chaque additif est fonction de sa nature et est déterminée facilement par l'homme de l'art.

La nature et la quantité d'additif sont choisies de façon à ne pas détruire la stabilité et le caractère fondant de la composition de l'invention. En particulier pour un alcool solide à 40°C tel que les alcools cétylique, stéarylique ou leurs mélanges, on utilise au moins 66% en poids d'alcool gras "liquide" par rapport au poids total d'alcool gras.

Le pH de la composition peut aller de 2 à 7 et de préférence de 2,5 à 4,5.

Selon l'invention, le milieu aqueux est par exemple constitué par de l'eau seule ou en mélange avec un solvant organique comme par exemple un alcool en C₁-C₄ tel que l'éthanol, le propylène glycol, le butylène glycol, l'isopropanol, un éther de glycol tel que les alkyl(C₁-C₄)éther de mono ou dipropylène glycol, mono ou diéthylène glycol, le dipropylène glycol, le diéthylène glycol et leurs mélanges.

Les compositions de l'invention sont utilisables plus spécialement dans le domaine cosmétique et plus particulièrement comme composition capillaire dont l'application est suivie éventuellement d'un rinçage à l'eau. Aussi, l'invention se rapporte à l'utilisation de cette composition comme ou pour la fabrication d'une composition à appliquer avant ou après tout traitement capillaire tel qu'un shampooing, une coloration ou une décoloration, une permanente ou un défrisage.

L'invention se rapporte également à l'utilisation de cette composition comme ou pour la fabrication d'une composition de coloration, de décoloration, de permanente ou de défrisage des cheveux.

L'invention a encore pour objet un procédé de traitement des fibres kératiniques consistant à appliquer sur lesdites fibres une composition telle que définie précédemment puis à effectuer éventuellement un rinçage à l'eau.

Les exemples qui suivent, donnés à titre illustratif et non limitatif, permettent de mieux faire ressortir les caractéristiques et avantages de l'invention.

Ces exemples portent sur des compositions après-shampooing à rincer contenant comme sel d'ammonium quaternaire un mélange M contenant 25% ± 5% en poids(par rapport au poids total des esters) de méthylsulfate d'acyloxyéthyl dihydroxyéthyl méthyl ammonium, 55% ± 5% de méthylsulfate de diacyloxyéthyl hydroxyéthyl méthyl ammonium et 20% ± 5% de méthylsulfate de triacyloxyéthyl méthyl ammonium, les radicaux acyles ayant de 14 à 18 atomes de carbone et provenant d'huile de palme partiellement hydrogénée. Ces esters sont en solution à 90% de matière active dans l'isopropanol.

Les compositions des exemples suivants sont par exemple obtenues en portant le mélange corps gras et sel d'ammonium à 70-80°C puis en ajoutant l'eau chauffée à la même température ; on agite ensuite vigoureusement avec une turbine pendant environ 10 minutes. On laisse ensuite refroidir sous agitation jusqu'à température ambiante.

### Exemple 1

On prépare une composition après-shampooing à rincer contenant :

| | |
|---|---|
| Mélange M | 15 gMA (Matière Active) |
| Alcool isostéarylique | 3 g |
| Eau | qsp 100 g |

Cette composition présente une viscosité avant manipulation de 1,3 Pa.s ; elle est "fondante" et se répartit parfaitement sur l'ensemble de la chevelure. Les cheveux traités avec cette composition sont doux, brillants et se démêlent facilement.

### Exemple 2

On reproduit l'exemple 1 en utilisant 6 g d'alcool isostéarylique au lieu de 3 g.

La composition a une viscosité avant manipulation de 3,4 Pa.s et présente les mêmes propriétés que celle de l'exemple 1.

### Exemple 3

On reproduit l'exemple 1 en remplaçant 3 g d'alcool isostéarylique par 6 g de 2-octyldodécanol.

La composition a une viscosité avant manipulation de 0,8 Pa.s et présente les mêmes propriétés que celle de l'exemple 1.

### Exemple 4

On reproduit l'exemple 1 en remplaçant 3 g d'alcool isostéarylique par 6 g d'alcool oléique.

La composition a une viscosité avant manipulation de 1,5 Pa.s et présente les mêmes propriétés que celle de l'exemple 1.

### Exemple 5

On reproduit l'exemple 1 en remplaçant 3 g d'alcool isostéarylique par 6 g d'alcool laurique oxyéthyléné à 4 moles d'oxyde d'éthylène.

La composition a une viscosité avant manipulation de 1,2 Pa.s et présente les mêmes propriétés que celle de l'exemple 1.

### Exemple 6

On reproduit l'exemple 1 en remplaçant 3 g d'alcool isostéarylique par 6 g d'heptaméthylnonane vendu sous la dénomination ARLAMOL HD par la société ICI.

La composition a une viscosité avant manipulation de 1,7 Pa.s et présente les mêmes propriétés que celle de l'exemple 1.

### Exemple 7

On reproduit l'exemple 1 en remplaçant 3 g d'alcool isostéarylique par 6 g de polydécène hydrogéné vendu sous la dénomination ETHYLFLO 366 NF par la société ETHYL CORPORATION.

La composition a une viscosité avant manipulation de 1,1 Pa.s et présente les mêmes propriétés que celle de l'exemple 1.

### Exemple 8

On reproduit l'exemple 1 en remplaçant 3 g d'alcool isostéarylique par 4 g d'alcool laurique.

La composition a une viscosité avant manipulation de 5,4 Pa.s et présente les mêmes propriétés que celle de l'exemple 1.

### Exemple 9

On reproduit l'exemple 1 en remplaçant 3 g d'alcool isostéarylique par 1 g d'alcool laurique.

La composition a une viscosité avant manipulation de 0,8 Pa.s et présente les mêmes propriétés que celle de l'exemple 1.

### Exemple 10

On prépare une composition après-shampooing à rincer contenant :

| | |
|---|---|
| Mélange M | 40 gMA |
| Alcool isostéarylique | 1 g |
| Eau | qsp 100 g |

Cette composition présente une viscosité de 11,3 Pa.s ; elle est fondante et se répartit parfaitement sur l'ensemble de la chevelure. Les cheveux traités avec cette composition sont doux, brillants et se démêlent facilement.

### Exemple 11

On prépare une composition après-shampooing à rincer contenant :

| | |
|---|---|
| Mélange M | 5 gMA |
| Alcool laurique | 9 g |
| Eau | qsp 100 g |

Cette composition présente une viscosité avant manipulation de 0,9 Pa.s ; elle est fondante et présente les mêmes propriétés que celle de l'exemple 1.

### Exemple 12

On prépare une composition après-shampooing à rincer contenant :

| | |
|---|---|
| Mélange M | 10 gMA |
| Alcool laurique | 12g |
| Eau | qsp 100 g |

Cette composition présente une viscosité de 5,9 Pa.s ; elle est fondante et présente les mêmes propriétés que celle de l'exemple 1.

### Exemples comparatifs 13, 14 et 15

On reproduit l'exemple 1 en remplaçant 3 g d'alcool isostéarylique par respectivement 3 g , 4,5 g et 6 g d'un mélange (70/30 en poids) d'alcools cétylique et stéarylique ( température de fusion 54°C ).

Bien que ces compositions présentent respectivement une viscosité avant manipulation de 0,7 Pa.s, de 2,2 Pa.s et de 5,2 Pa.s, elles ne sont pas fondantes.

### Exemples comparatifs 16,17 et 18

On reproduit l'exemple 1 en remplaçant 3 g d'alcool isostéarylique par 6 g d'huile de colza, 6 g d'huile de vaseline ou 6 g d'érucate d'octyldécyle.

Les compositions sont fluides, elles présentent une viscosité inférieure à 0,3 Pa.s.

### Exemple 19

On prépare une composition après-shampooing à rincer contenant :

| | |
|---|---|
| Mélange M | 15 gMA |
| Alcool isostéarylique | 4 g |
| Alcool cétylstéarylique (70/30 en poids ) | 2g |
| Eau | qsp 100 g |

Cette composition présente une viscosité avant manipulation de 2,2 Pa.s ; elle est fondante et présente les mêmes propriétés que celle de l'exemple 1.

### Test d'analyse sensorielle

Le caractère fondant a été apprécié par des experts selon une analyse sensorielle.

Un premier test a porté sur trois compositions A, B et C correspondant respectivement aux contre-exemple et exemples 14, 4 et 2.

Un deuxième test a été réalisé par les mêmes experts sur les mêmes compositions dénommées D, E, F.
La notation va de 0 (non fondant) à 5 (très fondant). Les moyennes des notes obtenues sont:

| | A | B | C |
|---|---|---|---|
| Fondant | 1,2 | 4,2 | 4,3 |
| | | | |

| | D(=A) | E(=B) | F(=C) |
|---|---|---|---|
| Fondant | 1,4 | 4,1 | 3,6 |

L'analyse statistique des données indique que les compositions B (et E) , C (et F) sont significativement supérieures à la composition A (=D) en terme de fondant au risque de 1/10 000.

## Revendications

1. Composition, caractérisée en ce qu'elle comprend dans un milieu aqueux, au moins un sel d'ammonium quaternaire comportant au moins une fonction ester, au moins un corps gras liquide à une température au plus égale à 40°C, le corps gras et les concentrations en sel d'ammonium et en corps gras étant choisis de façon que la composition ait une viscosité au moins égale à 0,6 Pa.s, au repos et avant utilisation et en ce qu'elle présente un caractère fondant.

2. Composition selon la revendication 1, caractérisée en ce que le sel d'ammonium quaternaire présente la formule ( I ) suivante : 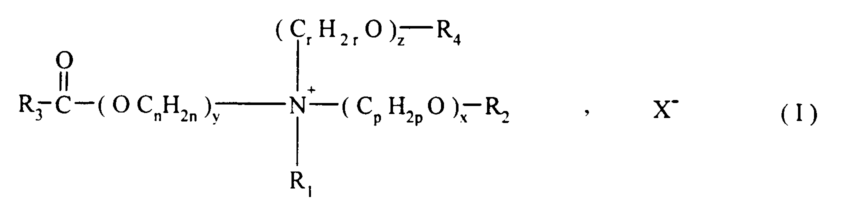 dans laquelle :
R₁ est choisi parmi les radicaux alkyles en C₁-C₆ et les radicaux hydroxyalkyles ou dihydroxyalkyles en C₁-C₆ ;
R₂ est choisi parmi :
- le radical 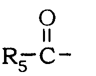
- les radicaux R₆ hydrocarbonés en C₁-C₂₂ linéaires ou ramifiés, saturés ou insaturés,
- l'atome d'hydrogène,
R₄ est choisi parmi :
- le radical 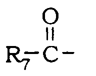
- les radicaux R₈ hydrocarbonés en C₁-C₆ linéaires ou ramifiés, saturés ou insaturés,
- l'atome d'hydrogène,
R₃, R₅ et R₇, identiques ou différents, sont choisis parmi les radicaux hydrocarbonés en C₇-C₂₁, linéaires ou ramifiés, saturés ou insaturés ;
n, p et r, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ;
à condition que la somme x + y + z vaille de 1 à 15 , que lorsque x vaut 0 alors R₂ désigne R₆ et que lorsque z vaut 0 alors R₄ désigne R₈.

3. Composition selon la revendication 2, caractérisée en ce que R₁ est choisi parmi les radicaux méthyle, éthyle, hydroxyéthyle et dihydroxypropyle.

4. Composition selon la revendication 2 ou 3, caractérisée en ce R₂ est un radical hydrocarboné long ayant de 12 à 22 atomes de carbone ou court ayant de 1 à 3 atomes de carbone.

5. Composition selon l'une quelconque des revendications 2 à 4, caractérisée en ce que R₄ est un radical hydrocarboné ayant 1 à 3 atomes de carbone.

6. Composition selon l'une quelconque des revendications 2 à 5, caractérisée en ce que R₃, R₅ et R₇, identiques ou différents, sont choisis parmi les radicaux hydrocarbonés en C₁₁-C₂₁, linéaires ou ramifiés, saturés ou insaturés.

7. Composition selon l'une quelconque des revendications 2 à 6, caractérisée en ce que x et z, identiques ou différents, valent 0 ou 1.

8. Composition selon l'une quelconque des revendications 2 à 7, caractérisée en ce que y est égal à 1.

9. Composition selon l'une quelconque des revendications 2 à 8, caractérisée en ce que n, p et r, identiques ou différents, valent 2 ou 3.

10. Composition selon l'une quelconque des revendications 2 à 9, caractérisée en ce que n, p et r sont égaux à 2.

11. Composition selon l'une quelconque des revendications 2 à 10, caractérisée en ce que la somme x+ y + z vaut de 1 à 10.

12. Composition selon l'une quelconque des revendications 2 à 11, caractérisée en ce que l'anion X⁻ est un halogénure, un alkylsulfate, le méthanesulfonate, le phosphate, le nitrate, le tosylate, un anion dérivé d'acide organique tel que l'acétate ou le lactate ou tout autre anion compatible avec l'ammonium quaternaire à fonction ester.

13. Composition selon l'une quelconque des revendications 2 à 12, caractérisée en ce que l'anion X⁻ est le chlorure ou le méthylsulfate.

14. Composition selon l'une quelconque des revendications 2 à 13, caractérisée en ce que R₁ désigne un radical méthyle ou éthyle ;
x et y sont égaux à 1 ;
z est égal à 0 ou 1 ;
n, p et r sont égaux à 2 ;
R₂ est choisi parmi :
- le radical 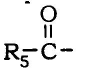
- les radicaux méthyle, éthyle ou hydrocarbonés en C₁₄-C₂₂
- l'atome d'hydrogène ;
R₄ est choisi parmi :
- le radical 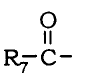
- l'atome d'hydrogène ;
R₃, R₅ et R₇, identiques ou différents, sont choisis parmi les radicaux hydrocarbonés en C₁₃-C₁₇, linéaires ou ramifiés, saturés ou insaturés.

15. Composition selon l'une quelconque des revendications 2 à 14, caractérisée en ce que les radicaux hydrocarbonés sont linéaires.

16. Composition selon l'une quelconque des revendications 2 à 15, caractérisée en ce que les composés de formule ( I ) sont choisis parmi les sels de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges, les radicaux acyles ayant de 14 à 18 atomes de carbone.

17. Composition selon l'une quelconque des revendications 1 à 16, caractérisée en ce qu'elle contient un mélange de sels de mono, di et triester d'ammonium quaternaire avec une majorité en poids de sels de diester.

18. Composition selon l'une quelconque des revendications 1 à 17, caractérisée en ce que le sel d'ammonium quaternaire est présent dans des quantités allant de 5% à 40% en poids par rapport au poids total de la composition.

19. Composition selon l'une quelconque des revendications 1 à 18, caractérisée en ce que le sel d'ammonium quaternaire est présent dans des quantités allant de 8% à 20% en poids par rapport au poids total de la composition.

20. Composition selon l'une quelconque des revendications 1 à 19, caractérisée en ce que le corps gras liquide est au moins un composé choisi parmi les alcools gras éventuellement oxyalkylénés ou polyglycérolés et les poly α-oléfines éventuellement hydrogénées et leurs mélanges.

21. Composition selon l'une quelconque des revendications 1 à 20, caractérisée en ce que le corps gras liquide est choisi parmi les alcools gras en C₈-C₂₂, linéaires ou ramifiés, éventuellement oxyalkylénés avec 1 à 15 moles d'oxyde d'alkylène ou polyglycérolés avec 1 à 6 moles de glycérol.

22. Composition selon la revendication 20, caractérisée en ce que les poly α-oléfines sont choisies parmi les polybutènes et les polydécènes éventuellement hydrogénés et leurs mélanges.

23. Composition selon l'une quelconque des revendications 1 à 21, caractérisée en ce que le corps gras liquide est choisi parmi les alcools laurique, myristique. isostéarylique, isocétylique et oléique, les alcools lauriques oxyéthylénés de 2 à 8 moles d'oxyde d'éthylène et leurs mélanges.

24. Composition selon l'une quelconque des revendications 1 à 23, caractérisée en ce que le corps gras liquide est présent dans des quantités allant de 1% à 15% en poids par rapport au poids total de la composition.

25. Composition selon l'une quelconque des revendications 1 à 24, caractérisée en ce que le corps gras liquide est présent dans des quantités allant de 2 % à 12% en poids par rapport au poids total de la composition.

26. Composition selon l'une quelconque des revendications 1 à 25, caractérisée en ce que la composition contient en outre au moins un additif choisi parmi les alcools gras solides à plus de 40°C, d'autres épaississants, les esters d'acides gras, les esters d'acides gras et de glycérol, les silicones, les tensioactifs cationiques autres que les sels d'ammonium quaternaire à fonction ester, les parfums, les conservateurs, les filtres solaires, les protéines, les vitamines, les polymères, les huiles végétales, animales, minérales ou synthétiques ou tout autre additif utilisé dans le domaine cosmétique.

27. Composition selon l'une quelconque des revendications 1 à 26, caractérisée en ce que le pH est compris dans un intervalle allant de 2 à 7.

28. Composition selon l'une quelconque des revendications 1 à 27, caractérisée en ce que le pH est compris dans un intervalle allant de 2,5 à 4,5.

29. Composition selon l'une quelconque des revendications 1 à 28, caractérisée en ce qu'elle consiste en une composition cosmétique.

30. Utilisation d'une composition selon l'une quelconque des revendications 1 à 29, comme, ou pour la fabrication d'une composition à appliquer avant ou après tout traitement capillaire tel qu'un shampooing, une coloration ou une décoloration, une permanente ou un défrisage des cheveux.

31. Utilisation d'une composition selon l'une quelconque des revendications 1 à 29, comme, ou pour la fabrication d'une composition de coloration, de décoloration, de permanente ou de défrisage des cheveux.

32. Procédé de traitement des fibres kératiniques, caractérisé par le fait que l'on applique sur lesdites fibres au moins une composition selon l'une quelconque des revendications 1 à 29.

## Claims

1. Composition, characterized in that it comprises, in an aqueous medium, at least one quaternary ammonium salt containing at least one ester function, at least one fatty substance which is liquid at a temperature of not more than 40°C, the fatty substance and the concentrations of ammonium salt and of fatty substance being chosen such that the composition has a viscosity at least equal to 0.6 Pa.s, at rest and before use, and in that it has a melting nature.

2. Composition according to Claim 1, characterized in that the quaternary ammonium salt has the formula (I) below: 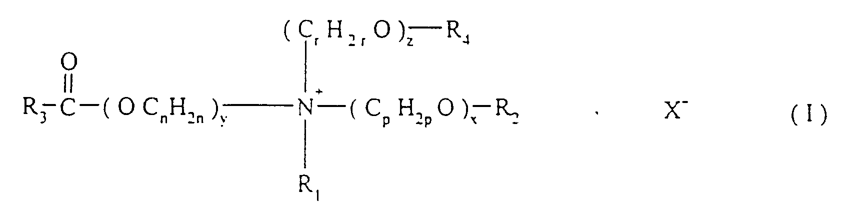 in which:
R₁ is chosen from C₁-C₆ alkyl radicals and C₁-C₆ hydroxyalkyl or dihydroxyalkyl radicals;
R₂ is chosen from:
- the radical 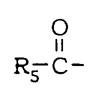
- linear or branched, saturated or unsaturated C₁-C₂₂ hydrocarbon radicals R₆,
- a hydrogen atom,
R₄ is chosen from:
- the radical 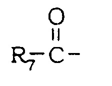
- linear or branched, saturated or unsaturated C₁-C₆ hydrocarbon radicals R₈,
- a hydrogen atom,
R₃, R₅ and R₇, which may be identical or different, are chosen from linear or branched, saturated or unsaturated C₇-C₂₁ hydrocarbon radicals;
n, p and r, which may be identical or different, are integers from 2 to 6;
y is an integer from 1 to 10;
x and z, which may be identical or different, are integers from 0 to 10;
X⁻ is a simple or complex, organic or inorganic anion;
on condition that the sum x + y + z is from 1 to 15, that when x is 0, then R₂ denotes R₆ and that when z is 0, then R₄ denotes R₈.

3. Composition according to Claim 2, characterized in that R₁ is chosen from the methyl, ethyl, hydroxyethyl and dihydroxypropyl radicals.

4. Composition according to Claim 2 or 3, characterized in that R₂ is a long hydrocarbon radical having from 12 to 22 carbon atoms or a short hydrocarbon radical having from 1 to 3 carbon atoms.

5. Composition according to any one of Claims 2 to 4, characterized in that R₄ is a hydrocarbon radical having 1 to 3 carbon atoms.

6. Composition according to any one of Claims 2 to 5, characterized in that R₃, R₅ and R₇, which may be identical or different, are chosen from linear or branched, saturated or unsaturated C₁₁-C₂₁ hydrocarbon radicals.

7. Composition according to any one of Claims 2 to 6, characterized in that x and z, which may be identical or different, are equal to 0 or 1.

8. Composition according to any one of Claims 2 to 7, characterized in that y is equal to 1.

9. Composition according to any one of Claims 2 to 8, characterized in that n, p and r, which may be identical or different, are equal to 2 or 3.

10. Composition according to any one of Claims 2 to 9, characterized in that n, p and r are equal to 2.

11. Composition according to any one of Claims 2 to 10, characterized in that the sum x + y + z is from 1 to 10.

12. Composition according to any one of Claims 2 to 11, characterized in that the anion X⁻ is a halide, an alkylsulphate, methanesulphonate, phosphate, nitrate, tosylate, an anion derived from an organic acid such as acetate or lactate or any other anion which is compatible with the quaternary ammonium containing an ester function.

13. Composition according to any one of Claims 2 to 12, characterized in that the anion X⁻ is chloride or methylsulphate.

14. Composition according to any one of Claims 2 to 13, characterized in that R₁ denotes a methyl or ethyl radical;
x and y are equal to 1;
z is equal to 0 or 1;
n, p and r are equal to 2;
R₂ is chosen from:
- the radical 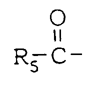
- methyl, ethyl or C₁₄-C₂₂ hydrocarbon radicals
- a hydrogen atom;
R₄ is chosen from:
- the radical 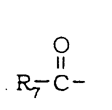
- a hydrogen atom;
R₃, R₅ and R₇, which may be identical or different, are chosen from linear or branched, saturated or unsaturated C₁₃-C₁₇ hydrocarbon radicals.

15. Composition according to any one of Claims 2 to 14, characterized in that the hydrocarbon radicals are linear.

16. Composition according to any one of Claims 2 to 15, characterized in that the compounds of formula (I) are chosen from diacyloxyethyldimethylammonium salts, diacyloxyethylhydroxyethylmethylammoniumsalts, monoacyloxyethyldihydroxyethylmethylammonium salts, triacyloxyethylmethylammonium salts, monoacyloxyethylhydroxyethyldimethylammonium salts and mixtures thereof, the acyl radicals having from 14 to 18 carbon atoms.

17. Composition according to any one of Claims 1 to 16, characterized in that it contains a mixture of quaternary ammonium mono-, di- and triester salts with a majority by weight of diester salts.

18. Composition according to any one of Claims 1 to 17, characterized in that the quaternary ammonium salt is present in amounts ranging from 5% to 40% by weight relative to the total weight of the composition.

19. Composition according to any one of Claims 1 to 18, characterized in that the quaternary ammonium salt is present in amounts ranging from 8% to 20% by weight relative to the total weight of the composition.

20. Composition according to any one of Claims 1 to 19, characterized in that the liquid fatty substance is at least one compound chosen from optionally oxyalkylenated or polyglycerolated fatty alcohols and optionally hydrogenated poly-α-olefins and mixtures thereof.

21. Composition according to any one of Claims 1 to 20, characterized in that the liquid fatty substance is chosen from linear or branched C₈-C₂₂ fatty alcohols optionally oxyalkylenated with 1 to 15 mol of alkylene oxide or polyglycerolated with 1 to 6 mol of glycerol.

22. Composition according to Claim 20, characterized in that the poly-α-olefins are chosen from optionally hydrogenated polybutenes and polydecenes, and mixtures thereof.

23. Composition according to any one of Claims 1 to 21, characterized in that the liquid fatty substance is chosen from lauryl, myristyl, isostearyl, isocetyl and oleyl alcohols, lauryl alcohols oxyethylenated with 2 to 8 mol of ethylene oxide, and mixtures thereof.

24. Composition according to any one of Claims 1 to 23, characterized in that the liquid fatty substance is present in amounts ranging from 1% to 15% by weight relative to the total weight of the composition.

25. Composition according to any one of Claims 1 to 24, characterized in that the liquid fatty substance is present in amounts ranging from 2% to 12% by weight relative to the total weight of the composition.

26. Composition according to any one of Claims 1 to 25, characterized in that the composition also contains at least one additive chosen from fatty alcohols that are solid above 40°C, other thickeners, fatty acid esters, fatty acid esters of glycerol, silicones, cationic surfactants other than the quaternary ammonium salts containing an ester function, fragrances, preserving agents, sunscreens, proteins, vitamins, polymers, plant, animal, mineral or synthetic oils or any other additive used in the cosmetic field.

27. Composition according to any one of Claims 1 to 26, characterized in that the pH is within the range from 2 to 7.

28. Composition according to any one of Claims 1 to 27, characterized in that the pH is within the range from 2.5 to 4.5.

29. Composition according to any one of Claims 1 to 28, characterized in that it consists of a cosmetic composition.

30. Use of a composition according to any one of Claims 1 to 29, as, or for the manufacture of, a composition to be applied before or after any hair treatment such as shampooing, dyeing, bleaching, permanent-waving or straightening of the hair.

31. Use of a composition according to any one of Claims 1 to 29, as, or for the manufacture of, a dyeing, bleaching, permanent-waving or straightening composition for the hair.

32. Process for treating keratin fibres, characterized in that at least one composition according to any one of Claims 1 to 29 is applied to the said fibres.

## Patentansprüche

1. Zusammensetzung, dadurch gekennzeichnet, daß sie in einem wässerigen Medium mindestens ein quaternäres Ammoniumsalz, das mindestens eine Estergruppe aufweist, und mindestens eine Fettsubstanz, die bei einer Temperatur von höchstens 40°C flüssig ist, enthält, wobei die Fettsubstanz und die Konzentrationen von Ammoniumsalz und Fettsubstanz so ausgewählt sind, daß die Zusammensetzung in Ruhe und vor der Verwendung eine Viskosität von mindestens 0,6 Pa·s aufweist, und dadurch, daß sie eine schmelzende Eigenschaft aufweist.

2. Zusammensetzung nach Anspruch 1, dadurch gekennzeichnet, daß das quaternäre Ammoniumsalz die folgende Formel (I) aufweist: 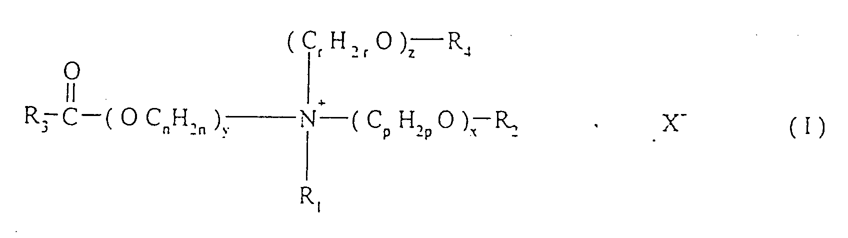 worin:
- R₁ unter den C₁₋₆-Alkylgruppen und den C₁₋₆-Hydroxyalkylgruppen oder den C₁₋₆-Dihydroxyalkylgruppen ausgewählt ist,
- R₂ ausgewählt ist unter:
- einer Gruppe 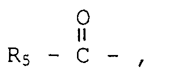
- den geradkettigen oder verzweigten, gesättigten oder ungesättigten C₁₋₂₂-Kohlenwasserstoffgruppen R₆ und
- Wasserstoff,
- R₄ ausgewählt ist unter:
- einer Gruppe 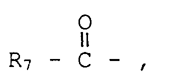
- den geradkettigen oder verzweigten, gesättigten oder ungesättigten C₁₋₆-Kohlenwasserstoffgruppen R₈ und
- Wasserstoff,
- R₃, R₅ und R₇, die identisch oder voneinander verschieden sind, unter den geradkettigen oder verzweigten, gesättigten oder ungesättigten C₇₋₂₁-Kohlenwasserstoffgruppen ausgewählt sind,
- n, p und r, die identisch oder voneinander verschieden sind, ganze Zahlen von 2 bis 6 bedeuten,
- y eine ganze Zahl von 1 bis 10 bedeutet,
- x und z, die identisch oder voneinander verschieden sind, Null oder ganze Zahlen von 1 bis 10 bedeuten und
- X⁻ ein einfaches oder komplexes, organisches oder anorganisches Anion bedeutet,
mit der Maßgabe, daß
- die Summe x + y + z 1 bis 15 ist,
- R₂ R₆ bedeutet, wenn x Null ist und
- R₄ R₈ bedeutet, wenn z Null ist.

3. Zusammensetzung nach Anspruch 2, dadurch gekennzeichnet, daß R₁ unter Methyl, Ethyl, Hydroxyethyl und Dihydroxypropyl ausgewählt ist.

4. Zusammensetzung nach Anspruch 2 oder 3, dadurch gekennzeichnet, daß R₂ eine langkettige Kohlenwasserstoffgruppe mit 12 bis 22 Kohlenstoffatomen oder eine kurzkettige Kohlenwasserstoffgruppe mit 1 bis 3 Kohlenstoffatomen ist.

5. Zusammensetzung nach einem der Ansprüche 2 bis 4, dadurch gekennzeichnet, daß R₄ eine Kohlenwasserstoffgruppe mit 1 bis 3 Kohlenstoffatomen ist.

6. Zusammensetzung nach einem der Ansprüche 2 bis 5, dadurch gekennzeichnet, daß R₃, R₅ und R₇, die identisch oder voneinander verschieden sind, unter den geradkettigen oder verzweigten, gesättigten oder ungesättigten Kohlenwasserstoffgruppen mit 11 bis 21 Kohlenstoffatomen ausgewählt sind.

7. Zusammensetzung nach einem der Ansprüche 2 bis 6, dadurch gekennzeichnet, daß x und z, die identisch oder voneinander verschieden sind, Null oder 1 bedeuten.

8. Zusammensetzung nach einem der Ansprüche 2 bis 7, dadurch gekennzeichnet, daß y gleich 1 ist.

9. Zusammensetzung nach einem der Ansprüche 2 bis 8, dadurch gekennzeichnet, daß n, p und r, die identisch oder voneinander verschieden sind, 2 oder 3 bedeuten.

10. Zusammensetzung nach einem der Ansprüche 2 bis 9, dadurch gekennzeichnet, daß n, p und r gleich 2 sind.

11. Zusammensetzung nach einem der Ansprüche 2 bis 10, dadurch gekennzeichnet, daß die Summe x + y + z 1 bis 10 ist.

12. Zusammensetzung nach einem der Ansprüche 2 bis 11, dadurch gekennzeichnet, daß das Anion X⁻ ein Halogenid, ein Alkylsulfat, Methansulfonat, Phosphat, Nitrat, Tosylat, ein von einer organischen Säure abgeleitetes Anion, wie Acetat oder Lactat, oder ein beliebiges weiteres Anion ist, das mit dem quaternären Ammoniumsalz mit Estergruppe kompatibel ist.

13. Zusammensetzung nach einem der Ansprüche 2 bis 12, dadurch gekennzeichnet, daß das Anion X⁻ Chlorid oder Methylsulfat ist.

14. Zusammensetzung nach einem der Ansprüche 2 bis 13, dadurch gekennzeichnet, daß
- R₁ Methyl oder Ethyl bezeichnet,
- x und y gleich 1 sind,
- z gleich Null oder 1 ist,
- n, p und r gleich 2 sind,
- R₂ ausgewählt ist unter:
- einer Gruppe 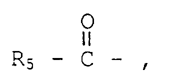
- Methyl, Ethyl oder einem C₁₄₋₂₂-Kohlenwasserstoff und
- Wasserstoff,
- R₄ ausgewählt ist unter:
- einer Gruppe 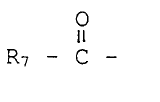 und
- Wasserstoff,
und
- R₃, R₅ und R₇, die identisch oder voneinander verschieden sind, unter den geradkettigen oder verzweigten, gesättigten oder ungesättigten C₁₃₋₁₇-Kohlenwasserstoffgruppen ausgewählt sind.

15. Zusammensetzung nach einem der Ansprüche 2 bis 14, dadurch gekennzeichnet, daß die Kohlenwasserstoffgruppen geradkettig sind.

16. Zusammensetzung nach einem der Ansprüche 2 bis 15, dadurch gekennzeichnet, daß die Verbindungen der Formel (I) unter den Diacyloxyethyldimethylammoniumsalzen, Diacyloxyethylhydroxyethylmethylammoniumsalzen, Monoacyloxyethyldihydroxyethylmethylammoniumsalzen, Triacyloxyethylmethylammoniumsalzen, Monoacyloxyethylhydroxyethyldimethylammoniumsalzen und den Gemischen dieser Salze ausgewählt sind, wobei die Acylgruppen 14 bis 18 Kohlenstoffatome aufweisen.

17. Zusammensetzung nach einem der Ansprüche 1 bis 16, dadurch gekennzeichnet, daß sie ein Gemisch aus quaternären Ammoniummono-, -di- und -triestersalzen enthält, wobei der Gewichtsanteil der Diestersalze überwiegt.

18. Zusammensetzung nach einem der Ansprüche 1 bis 17, dadurch gekennzeichnet, daß das quaternäre Ammoniumsalz in Mengenanteilen von 5 bis 40 Gew.-%, bezogen auf das Gesamtgewicht der Zusammensetzung, vorliegt.

19. Zusammensetzung nach einem der Ansprüche 1 bis 18, dadurch gekennzeichnet, daß das quaternäre Ammoniumsalz in Mengenanteilen von 8 bis 20 Gew.-%, bezogen auf das Gesamtgewicht der Zusammensetzung, vorliegt.

20. Zusammensetzung nach einem der Ansprüche 1 bis 19, dadurch gekennzeichnet, daß die flüssige Fettsubstanz mindestens eine Verbindung ist, die unter den Fettalkoholen, die gegebenenfalls alkoxyliert oder mit Glycerin verethert sind, und den gegebenenfalls hydrierten Poly-α-Olefinen sowie den Gemischen dieser Verbindungen ausgewählt ist.

21. Zusammensetzung nach einem der Ansprüche 1 bis 20, dadurch gekennzeichnet, daß die flüssige Fettsubstanz unter den geradkettigen oder verzweigten C₈₋₂₂-Fettalkoholen, die gegebenenfalls mit 1 bis 15 Mol Alkylenoxid alkoxyliert oder mit 1 bis 6 Mol Glycerin verethert sind, ausgewählt ist.

22. Zusammensetzung nach Anspruch 20, dadurch gekennzeichnet, daß die Poly-α-Olefine unter den Polybutenen und Polydecenen, die gegebenenfalls hydriert sind, sowie den Gemischen dieser Verbindungen ausgewählt sind.

23. Zusammensetzung nach einem der Ansprüche 1 bis 21, dadurch gekennzeichnet, daß die flüssige Fettsubstanz unter Laurylalkohol, Myristylalkohol, Isostearylalkohol, Isocetylalkohol und Oleylalkohol, den mit 2 bis 8 Mol Ethylenoxid ethoxylierten Laurylalkoholen und den Gemischen dieser Verbindungen ausgewählt ist.

24. Zusammensetzung nach einem der Ansprüche 1 bis 23, dadurch gekennzeichnet, daß die flüssige Fettsubstanz in Mengenanteilen von 1 bis 15 Gew.-%, bezogen auf das Gesamtgewicht der Zusammensetzung, vorliegt.

25. Zusammensetzung nach einem der Ansprüche 1 bis 24, dadurch gekennzeichnet, daß die flüssige Fettsubstanz in Mengenanteilen von 2 bis 12 Gew.-%, bezogen auf das Gesamtgewicht der Zusammensetzung, vorliegt.

26. Zusammensetzung nach einem der Ansprüche 1 bis 25, dadurch gekennzeichnet, daß die Zusammensetzung ferner mindestens einen Zusatzstoff enthält, der unter Fettalkoholen, die über 40°C fest sind, weiteren Verdickungsmitteln, Fettsäureestern, Estern von Fettsäuren und Glycerin, Siliconen, kationischen grenzflächenaktiven Stoffen, die von den quaternären Ammoniumsalzen mit Estergruppe verschieden sind, Parfums, Konservierungsmitteln, Sonnenschutzfiltern, Proteinen, Vitaminen, Polymeren, pflanzlichen, tierischen, mineralischen oder synthetischen Ölen oder beliebigen weiteren in der Kosmetik verwendeten Zusatzstoffen ausgewählt ist.

27. Zusammensetzung nach einem der Ansprüche 1 bis 26, dadurch gekennzeichnet, daß der pH-Wert in einem Bereich von 2 bis 7 liegt.

28. Zusammensetzung nach einem der Ansprüche 1 bis 27, dadurch gekennzeichnet, daß der pH-Wert in einem Bereich von 2,5 bis 4,5 liegt.

29. Zusammensetzung nach einem der Ansprüche 1 bis 28, dadurch gekennzeichnet, daß sie in einer kosmetischen Zusammensetzung besteht.

30. Verwendung einer Zusammensetzung nach einem der Ansprüche 1 bis 29 als Zusammensetzung zum Auftragen vor oder nach einer beliebigen Haarbehandlung, wie einer Haarwäsche, einer Färbung oder einer Entfärbung, einer Dauerwelle oder einer Entkräuselung der Haare, oder zur Herstellung einer solchen Zusammensetzung.

31. Verwendung einer Zusammensetzung nach einem der Ansprüche 1 bis 29 als Zusammensetzung zum Färben, Entfärben, Dauerwellen oder Entkräuseln der Haare, oder zur Herstellung einer solchen Zusammensetzung.

32. Verfahren zur Behandlung von Keratinfasern, dadurch gekennzeichnet, daß auf die Fasern mindestens eine Zusammensetzung nach einem der Ansprüche 1 bis 29 aufgetragen wird.
